# EUROPÄISCHE PATENTSCHRIFT

(11) **EP 2 399 620 B1**
(45) Veröffentlichungstag und Bekanntmachung des Hinweises auf die Patenterteilung: **10.08.2016**
(21) Anmeldenummer: 11168168.0
(22) Anmeldetag: 31.05.2011
(51) Int. Cl.: A61L 31/02, A61L 31/14

(54) **Implantat und Verfahren zur Herstellung desselben**
Implant and method for producing the same
Implant et son procédé de fabrication

(30) Priorität: 28.06.2010 US 358963 P
(43) Veröffentlichungstag der Anmeldung: 28.12.2011
(73) Patentinhaber: Biotronik AG, 8180 Bülach (CH)
(72) Erfinder: Bayer, Ullrich, 18211 Admannshagen-Bargeshagen (DE)
(74) Vertreter: Randoll, Sören

(56) Entgegenhaltungen:
- WO-A2-99/03515
- US-A1- 2006 229 711
- US-A1- 2008 281 396
- US-A1- 2009 017 087
- US-A1- 2010 076 544

## Beschreibung

Die vorliegende Erfindung betrifft ein Implantat, insbesondere eine intraluminale Endoprothese, dessen Körper zumindest überwiegend einen Werkstoff mit dem Hauptbestandteil Eisen aufweist, sowie ein Verfahren zur Herstellung eines entsprechenden Implantats.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, insbesondere Wire-Mesh-Stents, Befestigungselemente für Knochen, beispielsweise Schrauben, Platten oder Nägel, Marknägel, Spiralbündelnägel, Kirschnerdrähte, Drähte für Septumokkluder, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heute werden als Implantate besonders häufig Stents eingesetzt, die zur Behandlung von Stenosen (Gefäßverengungen) dienen. Sie weisen einen Körper in der Form eines ggf. durchbrochenen rohrförmigen oder hohlzylinderförmigen Gitters auf, das an beiden Längsenden offen ist. Das rohrförmige Gitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können. Ein Ansatz zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Der Körper kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren vorwiegend auf Legierungen von Magnesium und Eisen. Die vorliegende Erfindung bezieht sich vorzugsweise auf Implantate, deren Körper zumindest überwiegend aus einem biodegradierbaren Werkstoff mit dem Hauptbestandteil Eisen besteht, insbesondere einer Eisenbasislegierung (im Folgenden auch kurz: Eisenlegierung).

Bei der Realisierung biodegradierbarer Implantate wird angestrebt, die Degradierbarkeit entsprechend der angestrebten Therapie bzw. der Anwendung des jeweiligen Implantats (koronar, intracranial, renal etc.) zu steuern. Für viele therapeutische Anwendungen ist beispielsweise ein wichtiger Zielkorridor, dass das Implantat in einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen erleidet. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, die Durchlässigkeit des Gefäßes zu gewährleisten, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben aufweist.

Implantate mit einer Eisenlegierung, insbesondere eisenhaltige Stents, sind besonders kostengünstig und einfach herstellbar. Beispielsweise für die Behandlung von Stenosen verlieren diese Implantate allerdings ihre mechanische Integrität bzw. Stützwirkung erst nach einem vergleichsweise langen Zeitraum, d.h. erst nach einer Verweildauer in dem behandelten Organismus von ca. 2 Jahren. Dies bedeutet, dass sich der Kollapsdruck bei eisenhaltigen Implantaten für diese Anwendung über die Zeit zu langsam verringert.

Eine lange Verweildauer von Implantaten kann Komplikationen bei der weiteren Behandlung des Patienten hervorrufen, nämlich beispielsweise dann, wenn das in Auflösung befindliche Implantat aufgrund seiner ferromagnetischen Eigenschaften die Untersuchung des behandelten Patienten im Magnetresonanztomograph nicht erlaubt bzw. zu stark beeinflusst. Ferner kann ein über die notwendige Verweildauer hinausgehende Präsenz des Stents in der Gefäßwand dort zu mechanischen Irritationen führen, die ihrerseits eine Wiederverengung des behandelten Gefäßes zur Folge haben. Weiterhin kann bei orthopädischen Implantaten (z.B. bei Knochenplatten) die Bildung von neuer Knochensubstanz zu mechanischen Spannungen zwischen dem zu langsam degradierenden Implantat und der neuen Knochensubstanz führen. Dies erzeugt insbesondere bei Kindern und Heranwachsenden Knochendeformationen bzw. -fehlbildungen. Für derartige Anwendungen ist es daher wünschenswert, wenn das Einsatzgebiet von Implantaten, deren Körper zumindest überwiegend einen Werkstoff mit dem Hauptbestandteil Eisen aufweist, durch schnellere Degradation erweitert werden könnte.

Im Stand der Technik sind bereits unterschiedliche Mechanismen der Degradationskontrolle von Implantaten beschrieben worden. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material beruhen. Diese führen dazu, dass die Degradationszeit verlängert wird. So wird der Degradationsschutz durch verschieden zusammengesetzte Schutzschichten und durch definierte geometrische Abstände (Diffusionsbarrieren) zwischen dem Korrosionsmedium und dem Grundmaterial abgesichert. Weitere Lösungen auf dem Gebiet der gesteuerten Degradation rufen durch das Aufbringen von physikalischen Veränderungen (z.B. lokale Querschnittsveränderungen) der Stent-Oberfläche (z.B. lokal chemisch unterschiedlich zusammengesetzte Multilayer) Sollbrucheffekte hervor. Andere bisher beschriebene Methoden konzentrieren sich darauf, die Korrosionsvorgänge durch Einbringen oberflächennaher Defekte zu initiieren und im weiteren Verlauf durch die sich dabei bildende Erhöhung der realen Oberfläche eine Korrosionsverstärkung zu erzielen. Eine weitere Möglichkeit besteht darin, diese Effekte von vornherein mit der Erhöhung der ursprünglichen Oberflächenrauheitswerte zu verbinden und somit die Bindungsneigung von beispielsweise Chloriden derart zu erhöhen, sodass diese in Verbindung mit erhöhter Umgebungsfeuchte bzw. in vivo zu einer beschleunigten Korrosion führen. Mit den zuvor genannten Lösungen gelingt es jedoch meist nicht, die durch den Degradationsprozess eintretende Auflösung und die daraus resultierenden Stegbrüche in das geforderte Zeitfenster zu legen. Die Folge ist eine entweder zu früh oder zu spät einsetzende Degradation bzw. eine zu große Variabilität der Degradation des Implantats.

Aus den Druckschriften EP 0 923 389 B1 bzw. WO 99/03515 A2 oder WO 2007/124230 A1 sind Implantate bekannt, welche in vivo durch Korrosion degradierbar sind. Das Material der bekannten Implantate enthält Eisen als Hauptbestandteil sowie in einer bestimmten, vorgegebenen Konzentration Kohlenstoff. Der Nachteil dieser Legierungen besteht darin, dass das Zweistoffsystem aus Kohlenstoff und Eisen mit zunehmendem Kohlenstoffgehalt sehr stark an Duktilität verliert, ohne dass in gleichem Maße die Korrosionsbeständigkeit sinkt.

Aus der Druckschrift DE 10 2008 002 601 A1 ist ein Implantat mit einem Grundkörper bekannt, der ganz oder in Teilen aus einer biokorrodierbaren Eisenlegierung besteht. Hierbei hat die biokorrodierbare Eisenlegierung die Formel Fe-P, wobei ein Anteil von P an der Legierung 0,01 bis 5 Gew.% beträgt und Fe sowie herstellungsbedingte Verunreinigungen den auf 100 Gew.% verbleibenden Restanteil an der Legierung einnehmen. Nachteilig an der bekannten Legierung ist jedoch, dass mit erhöhtem P-Anteil die Duktilität des Materials sinkt und es somit schlechter verarbeitbar ist. Allerdings führt die Zugabe von P zu einer Härtesteigerung des Materials. Das in dem Dokument ebenfalls erwähnte Mn als Legierungsbestandteil dient als Hilfsmittel zur Ausscheidung von feinphasigen Pd-haltigen intermetallischen Verbindungen, welche ausschließlich durch das Zulegieren von Edelmetallen und/oder schweren Elementen erzeugt werden. Die Verwendung von Edelmetallen oder Schwermetallen ist jedoch häufig problematisch und kostentreibend.

In der Druckschrift DE 10 2004 036 954 A1 wird ein implantierbarer Körper für die intersomatische Fusion (spinale Fusion) bereitgestellt, der aus einem bioresorbierbaren, metallischen Werkstoff gefertigt ist. Der metallische Werkstoff weist als Hauptbestandteil Alkalimetalle, Erdalkalimetalle, Eisen, Zink oder Aluminium auf. Als Nebenbestandteile können Mangan, Cobalt, Nickel, Chrom, Kupfer, Cadmium, Blei, Zinn, Thorium, Zirconium, Silber, Gold, Palladium, Platin, Rhenium, Silicium, Calcium, Lithium, Aluminium, Zink, Kohlenstoff, Schwefel, Magnesium und/oder Eisen eingesetzt werden. Der Vorteil eines derartigen Materials besteht darin, dass der Werkstoff besonders günstige mechanische Eigenschaften, insbesondere im Hinblick auf Elastizität, Verformbarkeit und Stabilität bei geringer Masse aufweist. Die Degradation des Materials liegt jedoch noch nicht in dem gewünschten Zeitfenster.

US 2006/0229711 A1 betrifft degradierbare medizinische Geräte. US 2009/0017087 A1 offenbart Osseo-induktive Metallimplantate. US 2008/0281396 A1 beschreibt medizinische Geräte wie Führungsdrähte und Stents.

Weitere Beispiele zu Implantaten, welche aus einer bioabbaubaren Eisenlegierung bestehen, werden in den Druckschriften DE 197 31 021 A1 und WO 2007/082147 A2 offenbart.

In den genannten Druckschriften werden Eisenlegierungen mit Nickel und Chrom beschrieben, welche, insbesondere wenn sie nicht elektropoliert werden, Nickel-Ionen freisetzen. Die Nickel-Ionen führen nicht nur bei Nickel-Allergikern zu negativen inflammatorischen Effekten. Auch Chrom-Ionen können, wenn sie in einem mittleren Konzentrationsbereich bis 12 Gew.% vorliegen, einzeln oder auch mit anderen Schwermetallen herausgelöst werden und möglicherweise negative Zellreaktionen hervorrufen. Im Falle der Durchführung einer Elektropolitur wird zwar die Abgabe von Nickel-Ionen verringert, die Degradationszeit jedoch aufgrund der Bildung von Passivierungsschichten in unvorteilhafter Weise verlängert.

Folglich besteht die Aufgabe der vorliegenden Erfindung darin, ein Implantat zu schaffen, das eine Degradation des Implantats im gewünschten Zielkorridor aufweist, insbesondere in einem kürzeren Zeitraum, ohne dass ein nennenswerter Duktilitätsverlust auftritt. Dabei soll die Degradation zu einem kontrollierbaren Zeitpunkt stattfinden und auch kompliziert geformte Implantate mit den gewünschten Degradationseigenschaften ausgestattet werden können. Entsprechend besteht die Aufgabe der Erfindung außerdem darin, ein kostengünstiges Verfahren zur Herstellung eines derartigen Implantats anzugeben.

Die obige Aufgabenstellung wird gelöst durch ein Implantat, dessen Körper zumindest überwiegend einen Werkstoff mit dem Hauptbestandteil Eisen aufweist, wobei der Werkstoff als ersten Nebenbestandteil Schwefel mit einer Konzentration von mehr als 0,2 Gew.% und höchstens 1 Gew.%, vorzugsweise höchstens 0,5 Gew.%, enthält und als zweiten Nebenbestandteil mindestens ein Element der Gruppe enthält, welche Calcium, Mangan und Magnesium umfasst, wobei die Konzentration des zweiten Nebenbestandteils Calcium mindestens 0,1 Gew.% und höchstens 1 Gew.%, vorzugsweise mehr als 0,2 Gew.% und höchstens 0,5 Gew.%, die Konzentration des zweiten Nebenbestandteils Mangan mindestens 0,5 Gew.% und höchstens 3 Gew.%, und/oder die Konzentration des zweiten Nebenbestandteils Magnesium mindestens 0,3 Gew.% und höchstens 1 Gew.%, vorzugsweise höchstens 0,5 Gew.% beträgt.

Bei der vorliegenden Erfindung umfasst der Körper des Implantats mindestens einen Teil des Implantats, vorzugsweise den Hauptteil des Implantats, welcher die mechanische Integrität des Implantats bewirkt.
Unter dem Begriff Gefüge wird im Folgenden die Anordnung der Bestandteile von Feststoffen (Festkörpern), insbesondere die Anordnung der Kristallite (Körner), Poren, amorphen Bereiche und Korngrenzbereiche des Implantatkörpers verstanden.

Der Vorteil des erfindungsgemäßen Implantats mit dem Werkstoff der angegebenen Zusammensetzung besteht darin, dass Legierungselemente wie Mn, Mg und Ca in einer Fe-Matrix des Gefüges als starke Sulfidbildner dienen, welche innere Lokalelemente darstellen. Lokalelemente beschleunigen den Degradationsprozess auf etwa das 1,5-fache der Degradationsgeschwindigkeit des Werkstoffs, wenn dieser aus spannungsarm geglühtem Reineisen besteht. Als starke Sulfidbildner können die genannten Legierungselemente in einem bevorzugten Ausführungsbeispiel dann agieren, wenn sie sich während des Schmelzprozesses fein verteilen können und die Größe der Partikel begrenzt werden kann.

Der Gehalt dieser Legierungselemente in dem erfindungsgemäßen Werkstoff ist in vorteilhafter Weise so gewählt, dass diese in Verbindung mit Schwefel zwar die Korrosionsbeständigkeit des Werkstoffs wesentlich senken, die Mindestanforderungen an die mechanischen Eigenschaften, die an ein medizinisches Implantat gestellt werden, durch den Werkstoff jedoch erfüllt werden. Bewegen sich die Gehalte der genannten Elemente außerhalb der angegebenen Bereiche, verläuft entweder die Degradation nicht beschleunigt oder die mechanischen Eigenschaften des Werkstoffs (wie z.B. die Bruchdehnung) werden zu stark verändert.

Ebenso von Vorteil ist, dass bei dem erfindungsgemäßen Werkstoff der Gehalt an Schwermetallen (außer Mn) auf Werte ≤ 1 Gew.% begrenzt wird. So können unerwünschte Zellreaktionen aufgrund von Nickel und Chrom vermieden werden.

Ein weiterer Vorteil des erfindungsgemäßen Implantats besteht darin, dass aufwendige Oberflächenbehandlungsmaßnahmen zur Beschleunigung des Degradationsverhaltens nicht mehr benötigt werden. Diese können jedoch wahlweise trotzdem als unterstützende Maßnahmen für die Degradationsbeschleunigung zur Anwendung kommen. Ebenfalls von Vorteil ist, dass das Material des erfindungsgemäßen Implantats keine zytotoxischen Reaktionen im Körper des Behandelten hervorruft, da beispielsweise Nickel nicht als Legierungselement eingesetzt wird.

Ferner kann der Korrosionsprozess besser kalkuliert werden, da keine Gradientenschichten vorhanden sind und der Korrosionsverlauf bis zur Selbstauflösung als linear betrachtet werden kann. Die Degradation verläuft durch das gesamte Volumen des Werkstoffs gleichmäßig.

In einem bevorzugten Ausführungsbeispiel beträgt die Konzentration des zweiten Nebenbestandteils Calcium mehr als 0,2 Gew.% und höchstens 0,5 Gew.% und/oder die Konzentration des zweiten Nebenbestandteils Magnesium höchstens 0,5 Gew.%.

Bei der genannten Konzentration des jeweiligen zweiten Nebenbestandteils wird die Degradierbarkeit des Werkstoffs zwar herabgesetzt, die mechanischen Eigenschaften des Werkstoffs jedoch nicht wesentlich beeinflusst.

In einem weiteren bevorzugten Ausführungsbeispiel weist der Werkstoff des Implantat-Körpers zusätzlich Chrom als Nebenbestandteil auf, vorzugsweise mit einer Konzentration von 0,1 Gew.% bis 1 Gew.%, besonders bevorzugt mit einer Konzentration von 0,1 Gew.% bis 0,5 Gew.%. Die sich ebenfalls mit Chrom bildenden Sulfide stellen ebenfalls innere Lokalelemente dar. Chrom trägt zudem noch zur Kornfeinung bei, so dass insgesamt durch Zusatz von Chrom die Degradation des Werkstoffs einerseits weiter beschleunigt wird, andererseits die mechanischen Eigenschaften des Werkstoffs durch den Effekt der Kornfeinung günstig beeinflusst werden. Der Gehalt an Chrom ist jedoch nicht so hoch, dass er aufgrund des Herauslösens ggf. mit anderen Schwermetallen in signifikantem Umfang negative Zellreaktionen hervorruft.

Als Hauptbestandteil des Werkstoffs enthält dieser vorzugsweise mehr als 80 Gew.%, besonders bevorzugt mehr als 90 Gew.% Eisen. Eine Eisenbasis-Legierung ist besonders kostengünstig herstellbar.

Es ist weiter von Vorteil, wenn der Werkstoff des Implantat-Körpers zusätzlich Sauerstoff aufweist, vorzugsweise mit einer Konzentration von 0,05 Gew.% bis 2 Gew.%, besonders bevorzugt mit einer Konzentration von 0,05 Gew.% bis 0,9 Gew.%. Zusammen mit den oben angegebenen Nebenbestandteilen kann der Sauerstoff die Bildung von oxidischen und oxisulfidischen Partikeln im Gefüge des Werkstoffs bewirken, welche weitere innere Lokalelemente zur Beschleunigung der Degradation ausbilden.

In einer vorteilhaften Weiterbildung der Erfindung enthält der Werkstoff des Implantat-Körpers zusätzlich mindestens ein Element der Gruppe, welche Kohlenstoff, Phosphor, Vanadium, Silicium, Cer, Molybdän, Titan, Wolfram und Zirconium umfasst. Die oben genannten Legierungsbestandteile senken durch Bildung von weiteren intermetallischen Verbindungen die Korrosionsbeständigkeit des Werkstoffs weiter, wobei die Mindestanforderungen an die mechanischen Eigenschaften, die an das medizinische Implantat gestellt werden, weiterhin erfüllt werden. Dies ist dadurch begründet, dass die erzeugten Ausscheidungen eine zusätzliche Härtung des Materials bewirken. Die Zugabe von Vanadium führt sowohl zur Bildung festigkeitserhöhender und kornfeinender Vanadiumkarbide als auch zur Bildung von Vanadiumoxiden (Vanadium(III)-oxid, Vanadium(V)-oxid), die ebenfalls einen Beitrag zur Korrosionsbeschleunigung liefern. Insbesondere bei einer feindispersen Verteilung dieser intermetallischen Verbindungen werden Biokompatibilitätsprobleme des sonst als toxikologisch problematisch erachteten Verbindung V₂O₅ nicht beobachtet. Die Elemente Molybdän, Titan, Wolfram und Zirconium dienen vorwiegend der Festigkeitssteigerung des Werkstoffs.

Hierbei neigt insbesondere Phosphor auch bei hohen Abkühlgeschwindigkeiten des Werkstoffs während der Herstellung zur Mikroseigerung. Diese Mikroseigerungen (Zonen mit bis zu 4 Gew.% Phosphor) führen zu einer erhöhten Korrosionsanfälligkeit.

Vorteilhaft ist insbesondere, wenn die Konzentration des Kohlenstoffs mindestens 0,1 Gew.% und höchstens 0,5 Gew.%, vorzugsweise höchstens 0,3 Gew.%, und/oder die Konzentration des Phosphors mindestens 0,05 Gew.% und höchstens 0,5 Gew.%, vorzugsweise höchstens 0,3 Gew.%, und/oder die Konzentration des Vanadiums mindestens 0,1 Gew.% und höchstens 0,5 Gew.%, vorzugsweise höchstens 0,2 Gew.%, und/oder die Konzentration des Siliciums mindestens 0,05 Gew.% und höchstens 0,5 Gew.%, vorzugsweise höchstens 0,3 Gew.%, und/oder die Konzentration des Cers mindestens 0,05 Gew.% und höchstens 0,5 Gew.%, vorzugsweise höchstens 0,3 Gew.%, beträgt.

Die oben angegebenen Gehalte der Legierungsbestandteile, die sulfidische und oxidische sowie oxisulfidische Mikropartikel bilden, sind so gewählt, dass diese einerseits Ausscheidungshärtungseffekte bewirken, die zu ausreichend hohen Festigkeitseigenschaften führen, und dabei die Bruchdehnung durch ebenfalls vorhandene Kornfeinungseffekte nicht unter 15% absinken lassen. Diese Partikel bilden in Verbindung mit dem Mikroseigerungen bildenden Phosphor genügend innere Lokalelemente, welche bei korrosiver Beanspruchung zu einer gleichmäßigen und gegenüber konventionellen Eisenbasis-Legierungen beschleunigten Auflösung des Implantat-Körpers führen.

Der größte Anteil der Sulfid-, Oxid- oder Oxisulfid-Ausscheidungen liegt mit seiner Korngröße im Submikrometerbereich (mittlerer Durchmesser kleiner als 1 µm). Beispielsweise sind die Ausscheidungen als dunkle Punkte (1 bis 3 µm Partikelgröße) als Fe- und Mn-Sulfide, -Oxisulfide bzw. -Oxide mikroskopisch in Längs- und Querschliffen nach Ätzung mit 3%-iger alkoholischer Salpetersäure mit einem Gesamt-Flächeninhalt aller Punkte von kleiner als 3% der Fläche des jeweiligen Schliffes sichtbar. Die Ausscheidungen können bedingt durch die vorangegangene Verformung in zeilenartiger Vorzugsrichtung vorliegen. Die Dunkelfärbung ergibt sich durch das höhere Korrosionspotential dieser Ausscheidungen bzw. durch Löcher, die sich durch Herauslösen der Partikel während des Ätzprozesses bilden.

Die obige Aufgabenstellung wird ferner gelöst durch ein Verfahren zur Herstellung eines Implantats, dessen Körper zumindest überwiegend einen Werkstoff mit dem Hauptbestandteil Eisen aufweist, umfassend die folgenden Schritte:
a) Herstellung einer Schmelze mit einer oben angegebenen Werkstoff-Zusammensetzung,
b) Herstellung einer Bramme durch Abkühlung der Schmelze in einer entsprechenden Form in einer bestimmten, vorgegebenen Abkühlungsrate, und vorzugsweise Durchführung mindestens eines Warmumformschrittes zur Herstellung eines Halbzeugs,
c) Nachbearbeitung des Halbzeugs oder der Bramme, vorzugsweise mittels Laserschneiden, bis die gewünschte Form, insbesondere eine Gitterform, des Implantat-Körpers hergestellt ist.

Als Halbzeuge können beispielsweise Zylinder, Hohlzylinder, Platten, Quader, Hohlquader oder dergleichen, insbesondere dünnwandige Rohre für Stents, hergestellt werden.

Das angegebene Herstellungsverfahren ist sehr kostengünstig, da es nur wenige Schritte beinhaltet. Zudem sind die einzelnen Herstellungsschritte gut beherrschbar, so dass die individuellen Eigenschaften des Werkstoffs bzw. des Implantats einfach eingestellt werden können. Die Gefügeeigenschaften des Halbzeugs bzw. der Bramme und damit auch des daraus hergestellten Implantats werden im Wesentlichen während der Abkühlung der Schmelze hergestellt, der für die Herstellung des Halbzeugs ggf. notwendige Warmumformschritt ist für die Einstellung der Gefügeeigenschaften höchstens von untergeordneter Bedeutung.

Die in Schritt b) verwendete Abkühlungsrate beträgt im Temperaturbereich zwischen 1200°C und 700°C mindestens 50 K/min und höchstens 100 K/min. Der primäre Erstarrungsvorgang, der beim Abgießen in die Bramme abläuft, und somit die dabei vorhandene Abkühlrate werden vorrangig durch die Temperatur der Schmelze, der Temperatur der kalten Bramme, der Brammengeometrie und das Schmelzvolumen bestimmt. Die Abkühlbedingungen sind so zu wählen, dass keine ausgeprägten Entmischungszonen (Seigerungen) entstehen. Das bedeutet, dass die Schmelze den Bereich zwischen liquidus und solidus so rasch wie möglich, d.h. in wenigen Sekunden, passieren sollte. Die darauf folgende Abkühlung im festen Zustand sollte so schnell erfolgen, dass der Temperaturbereich zwischen 1200°C und 700°C in einem Zeitraum von maximal 10 Minuten durchschritten wird. Dies ist bei einem Brammendurchmesser von maximal 60 mm in der Regel gegeben.

Bei dem erfindungsgemäßen Herstellungsverfahren erfolgt die Abkühlung des Werkstoffs aus der Schmelze so rasch, dass die erzeugten Partikeln nicht agglomerieren, sondern sich fein im Gefüge verteilen. Dies hat einen positiven Einfluss auf die mechanischen Eigenschaften des resultierenden Implantats, insbesondere wird die Bruchdehnung nicht wesentlich verringert.

Um während des Abkühlprozesses entstandene Inhomogenitäten im Gefüge zu entfernen, kann die Bramme anschließend noch durch Spanen bzw. Abdrehen der Oberfläche bzw. durch Tieflochbohren eines Kerns bearbeitet werden. Durch diese Verfahrensweise wird sichergestellt, dass ausschließlich homogenes Gefüge aus dem mittleren Querschnitt der Bramme zur Weiterverarbeitung gelangt.

Der oben angegebene, fakultative, mindestens eine Warmumformschritt könnte beispielsweise Warmschmiedeverfahren, Rohrziehen mit Zwischenglühschritten oder dergleichen umfassen. Beispielsweise wird ein Rohr aus der Bramme durch mehrstufiges Warmum-formen oberhalb der Rekristallisationstemperatur hergestellt. Diese liegt bei der erfindungsgemäßen Legierung bei einer Temperatur oberhalb von 550°C und vorzugsweise unterhalb von 900°C. Durch den Temperatureinfluss erfährt das Material keine Kaltverfestigung, da bei Temperaturen zwischen 590°C und 630°C immer eine dynamische Rekristallisation erfolgt. Diese führt zur Bildung eines Rekristallisationsgefüges, das eine zwar eine geringe Festigkeit aber auch ein hohes Verformungsvermögen aufweist. Anschließend kann ein Tieflochbohren der Stange bei Raumtemperatur durchgeführt werden. Danach erfolgen weitere, beispielsweise 20, Ziehschritte bei Raumtemperatur oder bei einer Temperatur von bis zu von 100°C. Nach jedem Ziehschritt kann eine Zwischenglühung bei einer Temperatur von 600°C über einen Zeitraum von 30 min bis 60 min an Luft erfolgen, welche das Gefüge rekristallisieren lässt und welche die vor dem Ziehschritt vorhandene Verformungsfähigkeit des Werkstoffs wieder herstellt.

Eine bevorzugte Ausführungsform des erfindungsgemäßen Verfahrens beinhaltet, dass zwischen Schritt b) und c) ein Spannungsarmglühschritt durchgeführt wird, der vorzugsweise in einem Temperaturbereich von 570°C bis 590°C erfolgt, besonders bevorzugt über eine Haltezeit von 30 Minuten für ein Halbzeug in der Form eines dünnwandigen Rohrs. Bevorzugt besitzt ein solches Rohr eine maximale Wandstärke von etwa 200 µm. Bei einem derartigen Spannungsarmglühschritt verbleibt noch ein Rest an Kaltverfestigung im Material, die dadurch noch vorhandenen Restverspannungen des ferritischen Gitters begünstigen die Korrosion ebenfalls. Eine Verzunderung wird weitestgehend vermieden.

Der erfindungsgemäße Schritt c) kann außer Laserschneiden alternativ oder zusätzlich das mechanische Entgraten, Korundstrahlen und/oder Beizen in mineralischen Säuremischungen, beispielsweise in verdünnter Salpetersäure, beinhalten, so dass der Implantat-Körper die gewünschte Geometrie erhält und von Fertigungsrückständen wie Schlacke und Grat befreit wird.

Auf einen derart hergestellten Implantat-Körper kann zusätzlich eine Beschichtung, beispielsweise eine Beschichtung enthaltend eine pharmazeutisch aktive Substanz oder eine die Degradation noch weiter beschleunigende Beschichtung, aufgebracht werden.

Unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Umgebung des Implantats hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können beispielsweise aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel oder Sirolimus (bzw. dessen Derivaten), bestehen.

Das erfindungsgemäße Verfahren bzw. das erfindungsgemäße Implantat werden nachfolgend anhand von Beispielen erläutert. Dabei bilden alle beschriebenen Merkmale den Gegenstand der Erfindung, unabhängig von ihrer Zusammenfassung in den Ansprüchen oder deren Rückbezüge.

Es wird ein Werkstoff mit der folgenden Zusammensetzung (in Gew.%) eingewogen:
Mn: 0,5 bis 3,0
Cr: 0,1 bis 0,5
V: 0,1 bis 0,2
C: 0,1 bis 0,3
S: größer als 0,2 bis 0,5
P: 0,05 bis 0,3
Mg: 0,3 bis 0,5
O: max. 0,9
Se: max. 0,3
Ca: größer als 0,2 bis 0,5
Ce: max. 0,3
Fe: Rest

Eine Schmelze der oben genannten Werkstoffzusammensetzung wird in Brammen vergossen. Im Bereich von 1200°C bis 700°C werden die Brammen mit einer mittleren Abkühlungsrate von 50 K/min abgekühlt. Dabei werden Abkühlbedingungen durch das Brammenvolumen, den Brammenquerschnitt sowie von den Umgebungsbedingungen (Raumtemperatur und Luftumwälzung) bestimmt.

Im konkreten Fall sind die Abkühlbedingungen so, dass der Temperaturbereich zwischen 1200°C und 700°C auch im Inneren der Bramme in spätestens 3 h durchlaufen wird. Hierbei weist die Bramme eine Wanddicke von 600 mm auf, hat einen Innenquerschnitt von 60 mm x 60 mm und eine Tiefe von 500 mm und besteht aus Grauguss. Dieses Volumen von 1,8 dm³ hat ein Gewicht von ca. 14,0 kg. Unter Raumtemperaturbedingungen kühlt diese Menge Stahl in nicht zusätzlich gekühlten Brammen innerhalb von höchstens 10 Minuten von 1200°C auf 700°C ab. Das entspricht einer mittleren Abkühlrate von ca. 50 K/min. Danach erfolgt das Entformen und es schließt sich eine mehrstufige Warmverformung an. Dabei werden in mehreren Stufen Stangen mit einem Enddurchmesser von 25,4 mm hergestellt. Das mehrstufige Warmumformen erfolgt oberhalb der Rekristallisationstemperatur. Diese liegt bei der angegebenen Legierung bei einer Temperatur oberhalb von 550°C und unterhalb von 900°C. Das bedeutet, dass zunächst aus der Bramme mittels Warmschmieden eine Stange mit dem Außendurchmesser von ca. 25 mm und einer Länge von 0,5 m erzeugt wird. Durch den Temperatureinfluss erfährt diese Stange keine Kaltverfestigung, da bei Temperaturen zwischen 590°C und 630°C immer eine dynamische Rekristallisation erfolgt. Diese führt zur Bildung eines Rekristallisationsgefüges, das eine zwar eine geringe Festigkeit aber auch ein hohes Verformungsvermögen aufweist.

Nach Abkühlen der Stange auf Raumtemperatur erfolgt das Tieflochbohren der Stange. Dabei wird im Zentrum der Stange über eine Stangenlänge von 0,5 m eine durchgehende Bohrung mit einem Innendurchmesser von 12,7 mm erzeugt.

Das nun vorhandene Rohr wird anschließend in ca. 20 Ziehschritten auf die Endgeometrie von 2,00 mm Außendurchmesser bei einer Wanddicke von 200 µm gezogen. Dabei kommt die Technologie des Rohrzuges mit mitlaufender Stange zum Einsatz. Bei diesem Rohrziehverfahren wird der Innendurchmesser des gezogenen Rohres über den Außendurchmesser einer mitlaufenden Stange geformt. Dabei muss die Länge der einzuführenden Stange mindestens der Länge des fertig gezogenen Rohres entsprechen. Nach jedem Ziehschritt erfolgt eine Zwischenglühung bei einer Temperatur von 600°C über 30 min bis 60 min an Luft. Nach jedem bei Raumtemperatur oder bei einer Temperatur bis ca. 100°C durchzuführendem Ziehschritt wird das Rohr durch ein Walzverfahren von der Stange gelöst und abschließend abgezogen. Um Verschweißungen zwischen der mitlaufenden Stange und dem Rohrinnendurchmesser zu vermeiden, muss die Stange aus einem anderen Stahl als die erfindungsgemäße Legierung bestehen. Am besten eignet sich dafür ein hochlegierter Stahl wie z.B. der 1.4841 mit Cr, Ni und Si als Hauptlegierungselemente.

Nach dem Erreichen der finalen Rohrabmessungen (Außendurchmesser von 2 mm und Wandstärke von 0,200 mm) und dem Abziehen der mitlaufenden Stange wird das Rohr spannungsarm geglüht. Dies erfolgt bei einer Temperatur zwischen 570°C und 590°C über 30 min.

Aus dem so hergestellten Rohr werden nun mittels bekannter Laserschneidverfahren stent-ähnliche Geometrien geschnitten. Die Endkontur dieser Stents, die sich durch annähernd quadratische Stegquerschnitte von etwa 100 µm x 100 µm auszeichnet, wird danach durch abtragende Verfahren wie Beizen in Säuregemischen, Korundstrahlen und Elektropolieren erzeugt. Hierdurch werden auch Fertigungsrückstände wie Schlacke und Grat entfernt. Die Länge eines so hergestellten Stents kann zwischen 10 mm und 30 mm betragen. Stents für periphere Anwendungen können jedoch auch länger sein.

Das Gefüge der so erzeugten Stents ist ferritisch. Neben den Ferritkörnern sind auch Anteile von maximal 30% Perlit vorhanden. Die mittlere Korngröße beträgt ca. 25 µm. Dies entspricht einer Korngrößenkennzahl von 8 nach ASTM.

Die beschriebene Werkstoffzusammensetzung des so hergestellten Stents degradiert im Vergleich zu Reineisen mit 99,5% Eisenanteil unter Körpermilieubedingungen etwa 1,5 Mal schneller. Das bedeutet, dass ein Stent mit einem Stegquerschnitt von 100 µm x 100 µm nach etwa 18 Monaten vollständig abgebaut ist. Ein Stent aus Reineisen wäre erst nach 24 Monaten restlos degradiert.

Orthopädische Implantate wie z.B. winkelstabile Platten für die Osteosynthese von Kleinfragmenten des Humerus werden spanend aus einer oben beschriebenen Stange mittels Fräsen und Bohren hergestellt. Bei diesen Implantaten betragen die Wanddicken ca. 0,3 mm bis 0,5 mm. Aufgrund der im Vergleich zu Stents relativen Robustheit werden diese mittels Gleitschleifen entgratet. Eine Elektropolitur kann optional zum Zwecke einer weiteren Kantenverrundung herangezogen werden.

Die Degradation bei einem so hergestellten orthopädischen Implantat, wie z.B. einer winkelstabilen Platte für die Osteosynthese, ist ähnlich zu der des oben dargestellten Stents. Eine solche winkelstabile Platte degradiert je nach konkreter Geometrie und Implantationsort im Zeitraum zwischen 2 und 3 Jahren. Eine Platte aus Reineisen baut sich dagegen erst nach mindestens 4 Jahren restlos ab.

Aufgrund der bereits unter Normalbedingungen vorhandenen Korrosionsneigung der oben beschriebenen Legierung sind die oben dargestellten Stents und orthopädischen Implantate unverzüglich nach dem letzten Nassbehandlungsschritt in Isopropanol oder Aceton zu spülen und mittels Warmluft trocken zu blasen. Hierdurch wird eine vorzeitige Korrosion vermieden.

## Patentansprüche

1. Implantat, insbesondere intraluminale Endoprothese, dessen Körper zumindest überwiegend einen Werkstoff mit dem Hauptbestandteil Eisen aufweist, **dadurch gekennzeichnet, dass** der Werkstoff als ersten Nebenbestandteil Schwefel mit einer Konzentration von mehr als 0,2 Gew.% und höchstens 1 Gew.%, enthält und als zweiten Nebenbestandteil mindestens ein Element der Gruppe enthält, welche Calcium, Mangan und Magnesium umfasst, wobei die Konzentration des zweiten Nebenbestandteils Calcium mindestens 0,1 Gew.% und höchstens 1 Gew.%, die Konzentration des zweiten Nebenbestandteils Mangan mindestens 0,5 Gew.% und höchstens 3 Gew.% und/oder die Konzentration des zweiten Nebenbestandteils Magnesium mindestens 0,3 Gew.% und höchstens 1 Gew.% beträgt.

2. Implantat nach Anspruch 1, **dadurch gekennzeichnet, dass** die Konzentration des zweiten Nebenbestandteils Calcium mehr als 0,2 Gew.% und höchstens 0,5 Gew.% und die Konzentration des zweiten Nebenbestandteils Magnesium mindestens 0,3 Gew.% und höchstens 0,5 Gew.% beträgt.

3. Implantat nach einem der Ansprüche 1 bis 2, **dadurch gekennzeichnet, dass** der Werkstoff des Implantat-Körpers zusätzlich Chrom aufweist, vorzugsweise mit einer Konzentration von 0,1 Gew.% bis 1 Gew.%, besonders bevorzugt mit einer Konzentration von 0,1 Gew.% bis 0,5 Gew.%.

4. Implantat nach einem der vorhergehenden Ansprüche, **dadurch gekennzeichnet, dass** der Werkstoff des Implantat-Körpers überwiegend Eisen aufweist, vorzugsweise mehr als 80 Gew.%, besonders bevorzugt mehr als 90 Gew.% Eisen enthält.

5. Implantat nach einem der vorhergehenden Ansprüche, **dadurch gekennzeichnet, dass** der Werkstoff des Implantat-Körpers zusätzlich Sauerstoff aufweist, vorzugsweise mit einer Konzentration von 0,05 Gew.% bis 2 Gew.%, besonders bevorzugt mit einer Konzentration von 0,05 Gew.% bis 0,9 Gew.%.

6. Implantat nach einem der vorhergehenden Ansprüche, **dadurch gekennzeichnet, dass** der Werkstoff des Implantat-Körpers zusätzlich mindestens ein Element der Gruppe enthält, welche Kohlenstoff, Phosphor, Vanadium, Silicium, Cer, Molybdän, Titan, Wolfram und Zirconium umfasst.

7. Implantat nach Anspruch 6, **dadurch gekennzeichnet, dass** die Konzentration des Kohlenstoffs mindestens 0,1 Gew.% und höchstens 0,5 Gew.%, vorzugsweise höchstens 0,3 Gew.%, und/oder die Konzentration des Phosphors mindestens 0,05 Gew.% und höchstens 0,5 Gew.%, vorzugsweise höchstens 0,3 Gew.%, und/oder die Konzentration des Vanadiums mindestens 0,1 Gew.% und höchstens 0,5 Gew.%, vorzugsweise höchstens 0,2 Gew.%, und/oder die Konzentration des Siliciums mindestens 0,05 Gew.% und höchstens 0,5 Gew.%, vorzugsweise höchstens 0,3 Gew.%, und/oder die Konzentration des Cers mindestens 0,05 Gew.% und höchstens 0,5 Gew.%, vorzugsweise höchstens 0,3 Gew.%, beträgt.

8. Verfahren zur Herstellung eines Implantats, insbesondere einer intraluminalen Endoprothese, dessen Körper zumindest überwiegend einen Werkstoff mit dem Hauptbestandteil Eisen aufweist, umfassend die folgenden Schritte:
a) Herstellung einer Schmelze mit einer in einem der vorhergehenden Ansprüchen angegebenen Werkstoff-Zusammensetzung,
b) Herstellung einer Bramme durch Abkühlung der Schmelze in einer entsprechenden Form in einer bestimmten, vorgegebenen Abkühlungsrate, und vorzugsweise Durchführung mindestens eines Warmumformschrittes zur Herstellung eines Halbzeugs, wobei die verwendete Abkühlungsrate im Temperaturbereich zwischen 1200°C und 700°C mindestens 50 K/min und höchstens 100 K/min beträgt.
c) Nachbearbeitung des Halbzeugs oder der Bramme, vorzugsweise mittels Laserschneiden, bis die gewünschte Form des Implantat-Körpers hergestellt ist.

9. Verfahren nach Anspruch 8, **dadurch gekennzeichnet, dass** zwischen Schritt b) und Schritt c) ein Temperschritt durchgeführt wird, vorzugsweise in einem Temperaturbereich von 570°C bis 590°C, besonders bevorzugt über eine Haltezeit von 30 Minuten für ein Halbzeug in der Form eines dünnwandigen Rohrs.

10. Verfahren nach einem der Ansprüche 8 oder 9, **dadurch gekennzeichnet, dass** nach Schritt c) auf den Implantat-Körper eine Beschichtung, vorzugsweise eine Beschichtung enthaltend eine pharmazeutisch aktive Substanz, aufgebracht wird.

## Claims

1. An implant, in particular an intraluminal endoprosthesis, the body of which comprises at least predominantly a material with iron as a main constituent, **characterised in that** the material contains sulphur as first minor constituent with a concentration of more than 0.2 % by weight and not more than 1 % by weight, and contains as second minor constituent at least one element selected from the group comprising calcium, manganese and magnesium, the concentration of the second minor constituent calcium being at least 0.1 % by weight and not more than 1 % by weight, the concentration of the second minor constituent manganese being at least 0.5 % by weight and not more than 3 % by weight, and/or the concentration of the second minor constituent magnesium being at least 0.3 % by weight and not more than 1 % by weight.

2. The implant according to claim 1, **characterised in that** the concentration of the second minor constituent calcium is more than 0.2 % by weight and at the most 0.5 % by weight; and the concentration of the second minor constituent magnesium is at least 0.3 % by weight and at the most 0.5 % by weight.

3. The implant according to either one of claims 1 to 2, **characterised in that** the material of the implant body comprises, in addition, chromium, preferably with a concentration of 0.1 % by weight to 1 % by weight, particularly preferably with a concentration of 0.1 % by weight to 0.5 % by weight.

4. The implant according to any one of the preceding claims, **characterised in that** the material of the implant body comprises predominantly iron, and preferably contains more than 80 % by weight, and particularly preferably more than 90 % by weight of iron.

5. The implant according to any one of the preceding claims, **characterised in that** the material of the implant body comprises, in addition, oxygen, preferably in a concentration of 0.05 % by weight to 2 % by weight, particularly preferably in a concentration of 0.05 % by weight to 0.9 % by weight.

6. The implant according to any one of the preceding claims, **characterised in that** the material of the implant body contains in addition at least one element selected from the group comprising carbon, phosphorus, vanadium, silicon, cerium, molybdenum, titanium, tungsten and zirconium.

7. The implant according to claim 6, **characterised in that** the concentration of carbon is at least 0.1 % by weight and at the most 0.5 % by weight, preferably at the most 0.3 % by weight, and/or the concentration of phosphorus is at least 0.05 % by weight and at the most 0.5 % by weight, preferably at the most 0.3 % by weight, and/or the concentration of vanadium is at least 0.1 % by weight and at the most 0.5 % by weight, preferably at the most 0.2 % by weight, and/or the concentration of silicon is at least 0.05 % by weight and at the most 0.5%, preferably at the most 0.3 % by weight, and/or the concentration of cerium is at least 0.05 % by weight and at the most 0.5 % by weight, preferably at the most 0.3 % by weight.

8. A method for producing an implant, in particular an intraluminal endoprosthesis, the body of which comprises at least predominantly a material with iron as the main constituent, comprising the following steps:
a) producing a melt with a material composition specified in any one of the preceding claims;
b) producing a slab by cooling the melt in an adequate mould at a certain predetermined cooling rate and, preferably, carrying out at least one hot forming step for producing a semi-finished part, the used cooling rate in the temperature range between 1200°C and 700°C being at least 50 K/min and at the most 100 K/min; and
c) post-processing of the semi-finished part or slab, preferably by laser cutting, until the desired shape of the implant body is produced.

9. The method according to claim 8, **characterised in that**, between steps b) and c), a tempering step is carried out, preferably in a temperature range from 570°C to 590°C, particularly preferably over a holding time of 30 minutes for a semi-finished part in the form of a thin-walled tube.

10. The method according to either one of claims 8 or 9, **characterised in that**, subsequent to step c), a coating, preferably a coating containing a pharmaceutically active substance, is applied to the implant body.

## Revendications

1. Implant, notamment endoprothèse intraluminale, dont le corps présente au moins principalement un matériau avec pour composant principal le fer, **caractérisé en ce que** le matériau contient en tant que premier composant secondaire du soufre avec une concentration supérieure à 0,2 % en poids et maximale de 1 % en poids, et en tant que deuxième composant secondaire au moins un élément du groupe, lequel comprend le calcium, le manganèse et le magnésium, où la concentration du deuxième composant secondaire calcium est d'au moins 0,1 % en poids et au maximum de 1 % en poids, la concentration du deuxième composant secondaire manganèse est d'au moins 0,5 % en poids et au maximum de 3 % en poids et/ou la concentration du deuxième composant secondaire magnésium est d'au moins 0,3 % en poids et au maximum de 1 % en poids.

2. Implant selon la revendication 1, **caractérisé en ce que** la concentration du deuxième composant secondaire calcium est supérieure à 0,2 % en poids et au maximum de 0,5 % en poids et la concentration du deuxième composant secondaire magnésium est d'au moins 0,3 % en poids et au maximum de 0,5 % en poids.

3. Implant selon l'une des revendications 1 à 2, **caractérisé en ce que** le matériau du corps d'implant présente en outre du chrome, de préférence avec une concentration de 0,1 % en poids à 1 % en poids, de manière particulièrement préférée avec une concentration de 0,1 % en poids à 0,5 % en poids.

4. Implant selon l'une des revendications précédentes, **caractérisé en ce que** le matériau du corps d'implant présente principalement du fer, de préférence plus de 80 % en poids, de manière particulièrement préférée plus de 90 % en poids de fer.

5. Implant selon l'une des revendications précédentes, **caractérisé en ce que** le matériau du corps d'implant présente en outre de l'oxygène, de préférence avec une concentration de 0,05 % en poids à 2 % en poids, de manière particulièrement préférée avec une concentration de 0,05 % en poids à 0,9 % en poids.

6. Implant selon l'une des revendications précédentes, **caractérisé en ce que** le matériau du corps d'implant contient en outre au moins un élément du groupe, lequel comprend du carbone, du phosphore, du vanadium, du silicium, du cérium, du molybdène, du titane, du tungstène et du zirconium.

7. Implant selon la revendication 6, **caractérisé en ce que** la concentration du carbone est d'au moins 0,1 % en poids et au maximum de 0,5 % en poids, de préférence au maximum de 0,3 % en poids et/ou la concentration du phosphore est d'au moins 0,05 % en poids et au maximum de 0,5 % en poids, de préférence au maximum de 0,3 % en poids, et/ou la concentration du vanadium est d'au moins 0,1 % en poids et au maximum de 0,5 % en poids, de préférence au maximum de 0,2 % en poids, et/ou la concentration du silicium est d'au moins 0,05 % en poids et au maximum de 0,5 % en poids, de préférence au maximum de 0,3 % en poids, et/ou la concentration du cérium est d'au moins 0,05 % en poids et au maximum de 0,5 % en poids, de préférence au maximum de 0,3 % en poids.

8. Procédé de fabrication d'un implant, notamment une endoprothèse intraluminale, dont le corps présente au moins principalement un matériau avec pour constituant principal le fer, comprenant les étapes suivantes :
a) fabrication d'un produit fondu avec une composition de matériau indiquée dans l'une des revendications précédentes,
b) fabrication d'une brame par le refroidissement du produit fondu dans une forme correspondante selon une vitesse de refroidissement définie, prédéterminée et de préférence, exécution d'au moins une étape de formage à chaud pour la fabrication d'un produit semi-fini, où la vitesse de refroidissement utilisée se situe dans le domaine de température entre 1200 °C et 700 %°C à au moins 50 K/min et au maximum à 100 K/min.
c) post-traitement du produit semi-fini ou de la brame, de préférence en utilisant le découpage ou laser jusqu'à la forme désirée du corps d'implant est fabriquée.

9. Procédé selon la revendication 8, **caractérisé en ce qu'**entre l'étape b) et l'étape c), une étape de mise en température est réalisée, de préférence, dans un domaine de température de 570 °C à 590 °C, de manière particulièrement préférée sur une durée de maintien de 30 minutes pour un produit semi-fini sous la forme d'un tube à parois minces.

10. Procédé selon l'une des revendications 8 ou 9, **caractérisé en ce qu'**après l'étape c), un revêtement, de préférence un revêtement contenant une substance pharmaceutiquement active, est rapporté sur le corps d'implant.
